Europäisches Patentamt

European Patent Office

Office européen des brevets

(11) Publication number: **0 241 847 B1**

# EUROPEAN PATENT SPECIFICATION

(45) Date of publication of patent specification: **24.07.91**

(51) Int. Cl.5: **A61K 31/21,** A61K 31/74, A61K 47/00

(21) Application number: **87105142.1**

(22) Date of filing: **07.04.87**

The file contains technical information submitted after the application was filed and not included in this specification

(54) **Use of a carboxylic acid in pharmaceutical preparations intended for topical application.**

(30) Priority: **18.04.86 DE 3613187**

(43) Date of publication of application:
**21.10.87 Bulletin 87/43**

(45) Publication of the grant of the patent:
**24.07.91 Bulletin 91/30**

(84) Designated Contracting States:
**AT BE CH DE ES FR GB GR IT LI LU NL SE**

(56) References cited:
**EP-A- 0 052 404
EP-A- 0 131 927
LU-A- 81 902**

**CHEMICAL ABSTRACTS, vol. 103, 1985, page 351, abstract no. 59328m, Columbus, Ohio, US; & ES-A-518 878 (INDUSTRIAL FARMACEUTICA DE LEVANTE S.A.) 01-03-1984**

(73) Proprietor: **Heinrich Mack Nachf.
Postfach 2064
W-7918 Illertissen(DE)**

(72) Inventor: **Fries, Walter
Einsteinring 25
W-7918 Illertissen(DE)**

(74) Representative: **Lederer, Franz, Dr. et al
Lederer, Keller & Riederer Patentanwälte
Lucile-Grahn-Strasse 22
W-8000 München 80(DE)**

## Description

It is known that drug preparations to be applied externally and containing nitric acid esters such as nitroglycerine, isosorbide dinitrate, isosorbide-5-mononitrate may cause a yellow discolouration of white clothing.

As a matter of fact, this yellow discolouration can be removed again by means of decolorizers, however, this involves a time and effort consuming process. It would be better to avoid or at least reduce, such a yellow discolouration in the first place.

It has been found that the addition of carboxylic acids to drug preparations to be applied externally and containing nitric acid esters is suitable to substantially avoid such a yellow discolouration of clothing. Suitable are aliphatic or aromatic carboxylic acids which are skin-compatible when externally applied. Preferred are hydroxy carboxylic acids, especially lactic acid and citric acid.

Thus, the subject matter of the invention is the use of a skin-compatible aliphatic or aromatic carboxylic acid in pharmaceutical preparations containing nitric acid ester and intended for topical application for avoiding or reducing, discolouration of clothing. Furthermore, the subject matter of the invention is the use of a skin-compatible aliphatic or aromatic carboxylic acid for the production of pharmaceutical preparations containing nitric acid ester and intended for topical application, which cause no or reduced discolouration of clothing.

In LU-A-81 902 there is described a pharmaceutical preparation containing isosorbide dinitrate. According to the example of the specification the preparation may contain 0,33 kg/200 kg (=0,165 %) sorbic acid and 0,2 kg/200 kg (= 0,1 %) citric acid.

EP-A-131 927 also describes a pharmaceutical preparation containing isosorbide dinitrate which may contain further additives like sorbic acid in amounts below 1 % by weight.

Examples of suitable carboxylic acids in the present invention are as follows:

1. Aromatic carboxylic acids such as benzoic acid;
2. Aliphatic carboxylic acids and in particular:
   a) unsaturated dicarboxylic acids such as fumaric acid and maleic acid;
   b) saturated dicarboxylic acids such as oxalic acid, malonic acid and succinic acid;
   c) hydroxy monocarboxylic acids such as lactic acid;
   d) hydroxy dicarboxylic acids such as malic acid and tartaric acid;
   e) hydroxy tricarboxylic acids such as citric acid.

Mixtures of the above-cited acids can be used as well.

If, depending on the acid chosen, despite the addition a slight yellow discolouration occurs, it is then possible, however, to remove it without the use of decolorizers by means of normal washing.

The cited acids are mixed into preparations to be applied topically in the usual manner, such as creams, ointments, milk or sprays to be applied topically.

Their amount of use ranges between 1 and 10 weight percent, preferably between 1 and 6 weight percent related to the preparation. When determining the amount to be used attention has to be paid to the kind of acid, its effectiveness and its skin-compatibility.

Example 1:

10 g isosorbide dinitrate are, together with

5 g citric acid,

dissolved under agitation at room temperature in

27.5 g di-isopropyladipate,

27.5 g polyethylene glycol 400 and

30 g ethanol.

What is obtained is a solution which can be packed into a suitable receptacle with a dosing device, for instance, a spray-dosing pump, and can be used as a spray.

Example 2:

72 g oleic-acid ester of decyl alcohol,

6 g sorbitansesquioleate and

18 g mixture of higher saturated fatty alcohols common in galenics with fatty alcohol sulfates and emulsifying agents (Emulgade® F) are heated to 80°C for melting.

Under intensive agitation this oil phase is combined with

171 g demineralized water heated to 70°C, to which

5 g lactic acid was added under agitation.

The obtained emulsion is cooled down to 25°C and divided into two halves.

30 g fine-crystalline isosorbide dinitrate with a grain size of below 100 $\mu$m are suspended in the one half of the cream by means of a high-speed agitator and are subsequently homogenized.

The remaining half of the cream is subsequently added to this concentrate, again intensively agitated at room temperature and finally homogenized.

Example 2, without the addition of lactic acid, corresponds to a preparation according to DE-A 3 325 466.

Obtained is a cream which can be packed into a receptacle with an ointment or cream dosing pump.

## Claims

1. Use of a skin-compatible aliphatic or aromatic carboxylic acid for the preparation of nitric acid ester containing pharmaceutical preparations for topical administration in an amount of 1-10% by weight of the preparation which is sufficient for said preparations to cause no or decreased discolouration of the clothing.

2. Use of a skin-compatible aliphatic or aromatic carboxylic acid in nitric acid ester containing pharmaceutical preparations for topical application for avoiding or decreasing discolouration of clothing in an amount of 1 - 10 % by weight of the preparation.

3. Use according to claim 1 or 2 characterized in that the carboxylic acid is used in an amount of 1-6 % by weight based on the preparation.

4. Use according to claim 1 or 2 characterized in that the nitric acid ester is nitroglycerine, isosorbide dinitrate or isosorbide-5-mononitrate.

5. Use according to claim 1 or 2 characterized in that the carboxylic acid is a hydroxy carboxylic acid.

6. Use according to claim 5 characterized in that the carboxylic acid is lactic or citric acid.

## Revendications

1. Utilisation d'un acide carboxylique aliphatique ou aromatique compatible avec la peau pour l'obtention de préparations pharmaceutiques contenant un ester d'acide nitrique, destinées à une administration topique en une quantité de 1 à 10 % en poids de préparation, suffisante pour que lesdites préparations ne produisent pas de changement de couleur ou ne produisent qu'un changement limité de couleur de l'étoffe.

2. Utilisation d'un acide carboxylique aliphatique ou aromatique compatible avec la peau dans des préparations pharmaceutiques contenant un ester d'acide nitrique pour l'application topique afin d'éviter ou de réduire le changement de couleur de l'étoffe, en une quantité de 1 à 10 % en poids de la préparation.

3. Utilisation suivant la revendication 1 ou 2, caractérisée en ce que l'acide carboxylique est utilisé en une quantité de 1 à 6 % en poids sur la base de la préparation.

4. Utilisation suivant la revendication 1 ou 2, caractérisée en ce que l'ester d'acide nitrique est la nitroglycérine, le dinitrate d'isosorbide ou le 5-mononitrate d'isosorbide.

5. Utilisation suivant la revendication 1 ou 2, caractérisée en ce que l'acide carboxylique est un acide hydroxycarboxylique.

6. Utilisation suivant la revendication 5, caractérisée en ce que l'acide carboxylique est l'acide lactique ou citrique.

## Patentansprüche

1. Verwendung einer hautverträglichen aliphatischen oder aromatischen Carbonsäure zur Herstellung von salpetersäureesterhaltigen pharmazeutischen Präparaten für topische Verabreichung in einer Menge von 1 bis 10 Gew.% des Präparates, die ausreichend ist, damit die Präparate keine oder eine verminderte Verfärbung der Kleidung bewirken.

2. Verwendung einer hautverträglichen aliphatischen oder aromatischen Carbonsäure in salpetersäureesterhaltigen pharmazeutischen Präparaten für topische Verabreichung zum Verhindern oder Vermindern der Verfärbung der Kleidung in einer Menge von 1 bis 10 Gew.% des Präparates.

3. Verwendung nach Anspruch 1 oder 2, dadurch gekennzeichnet, daß die Carbonsäure in einer Menge von 1 bis 6 Gew.%, bezogen auf das Präparat, verwendet wird.

4. Verwendung nach Anspruch 1 oder 2, dadurch gekennzeichnet, daß der Salpetersäureester Nitroglycerin, Isosorbiddinitrat oder Isosorbid-5-mononitrat ist.

5. Verwendung nach Anspruch 1 oder 2, dadurch gekennzeichnet, daß die Carbonsäure eine Hydroxycarbonsäure ist.

6. Verwendung nach Anspruch 5, dadurch gekennzeichnet, daß die Carbonsäure Milch- oder Citronensäure ist.